# EUROPEAN PATENT SPECIFICATION

(11) **EP 0 799 067 B1**
(45) Date of publication and mention of the grant of the patent: **08.08.2001**
(21) Application number: 95942340.1
(22) Date of filing: 19.12.1995
(51) Int. Cl.: A61M 15/00

(54) **AN INHALATION DEVICE, A METHOD OF DISPERSING A PHARMACEUTICALLY ACTIVE SUBSTANCE AND A METHOD OF ADMINISTERING A DOSE OF A PHARMACEUTICALLY ACTIVE SUBSTANCE**
INHALIERVORRICHTUNG, VERFAHREN ZUM VERTEILEN VON PHARMAZEUTISCH AKTIVEN SUBSTANZEN UND EIN VERFAHREN ZUM VERABREICHEN VON DOSEN VON PHARMAZEUTISCH AKTIVEN SUBSTANZEN
DISPOSITIF D'INHALATION, PROCEDE DE DISPERSION D'UN PRODUIT A EFFICACITE PHARMACEUTIQUE ET PROCEDE D'ADMINISTRATION D'UNE DOSE D'UN PRODUIT A EFFICACITE PHARMACEUTIQUE

(30) Priority: 21.12.1994 SE 9404439
(43) Date of publication of application: 08.10.1997
(73) Proprietor: AstraZeneca AB, 151 85 Södertälje (SE)
(72) Inventor: WETTERLIN, Kjell, S-247 34 Södra Sandby (SE)
(86) International application number: PCT/SE95/01539
(87) International publication number: WO 96/19253

(56) References cited:
- US-A- 5 341 801

## Description

The present invention relates to an inhalation device for inhalation of a pharmaceutically active substance mounted on an inhaler, as described in claim 1.

The inhalation device according to the invention is preferably mounted on a breath-actuated dry-powder inhaler, containing multiple doses of a medicament containing an active substance, the inhaler having manoeuvring unit comprising a manoeuvring unit for loading one dose of the medicament to a dosing unit and providing said dose in a position for inhalation. An inhaler of the prescribed type is described in EP-A-0 069 715 and EP-A-0 237 507.

The device according to the invention is especially designed for patients who are not able to actively inhale or who are not able to create the inhalation flow necessary to release and lift the dose of the substance into the inhalation channel and to the lungs when using a breath-actuated inhaler.

### Background of invention

Inhalable pharmaceutically active substances are generally used for treatment of diseases in the bronchial and pulmonary area, such as asthma and chronical bronchitis. Various embodiments of inhalation devices or apparatus are used for the purpose. The function of these known devices is depending on the creation of an airflow through the inhalation device caused by an inhalation by the patient. The airflow causes active substance to moved from a release position into the airflow in which it is dispersed. A specially advantageous inhaler of the above mentioned type is the dry-powder, breath-actuated multidose inhaler Turbuhaler®, schematically described in the above mentioned EP-patents.

Some patients such as small children and elderly people with diseases in the bronchial area are not able to use a breath-actuated inhaler as it might be hard or even impossible for these patients to achieve the necessary inhalation flow and these patient are today reduced to the use of inhalers using pressurised gas, i.e. freon. Such inhalers suffer from many known disadvantages, such as unwanted side effects.

Furthermore, it is presently a problem to administer an Inhalable substance to an asthma patient who is anaesthetised during an operation and the patient can not actively inhale. For many asthmatic patients the administration of asthma pharmaceuticals during an operation is vital.

### Prior art

In order to facilitate the inhalation of pharmaceutically active substances being administered by the use of pressurised metered dose inhalers, so called pMDI:s, it is known to provide expansion chambers into which the substance, with the pressurised gas, is dispersed. These devices are generally called spacers and a typical spacer is known from GB 1 565 029.

Furthermore, inhalation devices including dispersion chambers have been developed for breath-actuated dry-powder inhalers of the above mentioned type. Such an inhalation device is described in EP-A-0 548 152. This device is however bulky and contains several mechanical parts which makes the device complicated and expensive to produce and to use. The reliability is not very high due to the complexibility of the device.

The present invention relates to an inhalation device of the above mentioned type which can be used by patients having reduced ability to create an inhalation flow necessary to lift the dose from the release position into the inhalation channel when using a breath-actuated inhaler, and which can be used to administer Inhalable substances to a patient being anaesthetised.

The invention provides a device which facilitates the use of especially a Turbuhaler® for patients presently being reduced to the use of pressurised metered-dose inhalers.

The inhalation device according to the invention has a non-complicated construction with few mechanical parts, is simple and cheap to produce and is easy to use by the patient.

In the device according to the invention a first non-movable element is fixed on the inhaler so that a second element will move in relation to both the first element and the inhaler when the device is activated for inhalation, as described in claim 1.

Further advantages with the present invention are clear form the depending claims 2 to 17.

The present invention also includes a method of dispersing a pharmaceutically active substance, in a dispersing chamber by creating a negative pressure or vacuum is in said dispersing by using a device as according to the invention. The dispersed substance could thereby be inhaled using an ordinary inhalation flow or it could be pressed out from the inhalation device, as described in claims 18 to 21.

### Brief description of the drawing

The inhalation device according to the present invention will now be described by way of example with reference to the appended drawings, in which
Fig. 1 shows a schematic view of a section of the inhalation device according to a first embodiment of the invention; and

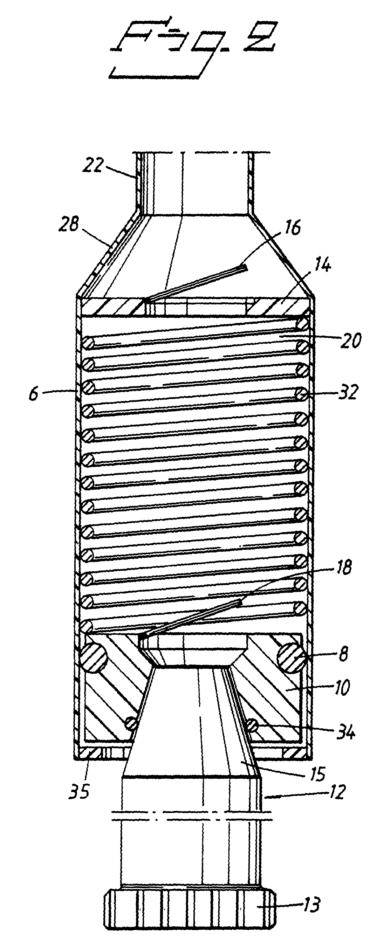

Fig. 2 shows a schematic view of a section of the second embodiment of the inhalation device according to the invention.

### Detailed description of the drawing

The device according to the invention is intended to be used in connection with an inhaler for inhalation of a pharmaceutically active substance, e.g. the breath-actuated, dry-powder, multidose inhaler 12 sold under the trademark Turbuhaler®. The inhalation device according to the invention may be modified within the scope of the appended claims to be used with any dry-powder inhaler which when activated positions a dose of the medicament in a release position in the inhalation channel.

The preferred inhaler 12 is provided with a reservoir for storing the substance, a metering or dosing unit, an air flow path having an air inlet and an air outlet. The inhaler is also provided with operating means comprising a manoeuvring unit 13 for moving a metering device from a loading position in which a predetermined dose of the substance to be inhaled is metered into the metering device and a release position where the dose is placed in the inhalation channel, released and carried by the inhalation air through the channel to the air outlet or mouthpiece 15 of the inhaler.

As can be seen in the drawings the inhalation device according to the invention comprises a first substantially non-moving element 10 which is provided on the air outlet or mouth piece 15 of the inhaler. The first element 10 is formed as a piston having an opening 26 which, when the piston is arranged on the air outlet of the inhaler, coincides with the opening 11 of the air outlet. The connection between the air outlet or mouth piece 15 of the inhaler and the piston is air tight through a sealing or as in the preferred embodiment the piston is rigidly mounted on the outer walls of the air outlet or mouth piece 15 of the inhaler in order to prevent air from entering between the two parts. The piston could be glued, welded or rigidly fastened in any other manner to the inhaler.

Around said first substantially non-moving element, i.e. the piston 10, a movable second element 6 is provided. Said second element 6 is hollow and substantially formed as a cylinder. In the upper part of the cylinder the walls merge and define a cone-shaped part 28. Above this cone-shaped part 28 a further cylinder 22 having a smaller diameter than the cylinder 6 defining the second element is arranged. Said cylinder 22 defines the air outlet of the dispersing chamber 20 and the inhalation device and is formed as a mouth piece or nose adapter part.

The piston 10 is arranged inside the cylinder 6. The piston thereby defines the bottom of the second element 6. A dispersing chamber 20 is defined in the cylinder 6 above the piston 10. Sealing means 8 are provided between the first and second elements at their mutual area of connection. The sealing means 8 are preferably provided as an O-ring sealing member and could be arranged in a groove provided on the outer surface of the piston.

As mentioned above the piston is formed with an opening 26 which coincides with the opening 11 of the air outlet or mouth piece 15 of the inhaler. First valve means 18 are provided regulating the air flow through the air outlet and the opening in the piston to the dispersing chamber. The valve means are provided as a one way valve arranged to only open when the air flows from the inhaler to the dispersing chamber. The valve could be of any known type and is in the preferred embodiment formed as a thin membrane of which one part is fixed to the wall of the piston and the other end is free moving.

The cylinder 6 is formed with a limiting wall member 14 having an outlet opening coinciding with the air outlet of the inhaler. The wall member 14 defines the upper restriction of the dispersing chamber 20 and is provided with holding means 30 for holding the inhaler in a fixed position in relation to the rotation when the manoeuvring unit 13 are rotated. These means could be provided as a ratchet mechanism, as teeth rings or have any other construction. Second valve means 16 are provided in said opening for regulating the air flow out from the dispersing chamber upon inhalation of a user. A mouth piece 22 or nose adapter is provided at the end of the cylinder 6.

The first and second valves 18 and 16 are preferably of similar construction and must be sensitive and easy to open in order to minimise the inhalation flow resistance and retention of substance within the inhalation device. For this purpose the valve could preferably be made as thin membranes of plastic or the like. The membranes could be fixed at one end in the opening of the piston 10 and the wall member 14 respectively, as can be seen in the figure. The wall member 14 acts as a valve seat for the valve 18.

In a first preferred embodiment a further hollow cylinder element 4 is provided. The cylinder is defined by a bottom and wall parts and is opened in its upper region. The bottom of said further cylinder is provided with air inlet openings 5 communicating with the inside of said further cylinder element 4. A mounting element 24 for the inhaler is provided inside said further cylinder and fixed to the bottom of the cylinder. The mounting element 24 comprises a portion 23 adapted to the form of the manoeuvring unit 13 of the inhaler in order for it to be fixedly mounted in said mounting means 24.

Due to this fixed and rigid mounting of the manoeuvring unit 13 of the inhaler 12 in the mounting part 23 the unit 13 is rotated an angle proportional to the rotation of the outer cylinder-formed element 4. The rotation of the outer cylinder-formed element 4 and thereby the manoeuvring unit 13 activates the inhaler 12 for inhalation as the manoeuvring unit places the dosing unit and thereby a dose in the release position within the inhalation channel.

The function of this embodiment will now be described.

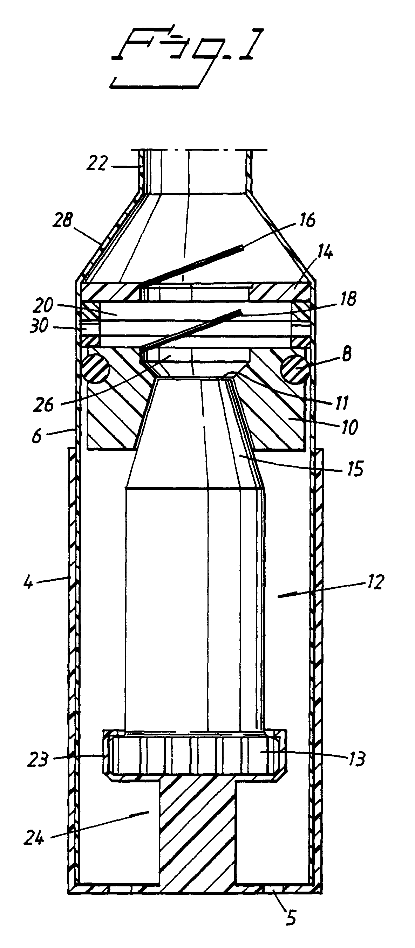

In the inactive position, which is shown in fig. 1, the inner cylinder-formed element 6 is totally interposed into the outer cylinder-formed element 4. When the outer element 4 is turned the manoeuvring unit 13 and the dosing unit of the inhaler is turned and a dose is placed in release position in the inhalation channel. The two cylinder formed element 4 and 6 are thereafter moved axially in relation to each other whereby the inhaler 12 with the piston 10 is pressed downwards along the inner wall of the inner element 6. A low-pressure or vacuum is created in the inner chamber 20 which is created when the elements are moved away from each other. The second valve 16 closes.

The valve 18 opens and air is drawn into the inhalation device through the air inlets 5 in the bottom part of the outer element 4. When the air passes the inhaler, the dose placed in the inhalation channel is released and dispersed into the inner chamber 20.

The dispersed substance could now be actively inhaled by the patient, a method which could be used also by small children, elderly people and others with reduced inhalation capacity .

Alternatively the substance can be forced out of the inner chamber by pressing the piston 10 and the second element 6 together again after the activation and dispersion of the dose has taken place. The volume in the dispersing chamber will then decrease and an over pressure or positive pressure will be created. The valve 16 will open and air/substance contents of the dispersing chamber will be forced out through the mouth piece 22 of the device. In this manner substance can be forced down into the lungs of the patient. This active pressing out of the substance is especially intended to be used when treating an anaesthetised patient but could also be used under other circumstances, e.g. by small children or elderly people refusing to inhale.

The inhalation device could also be constructed without the outer cylinder 4.

A biasing element 32 is thereby provided between the piston 10, which is mounted on the mouthpiece and/or upper part of the inhaler, and the cylinder 6. The cylinder 6 is provided with stop means 35 provided at the opened end of the cylinder preventing the cylinder from being separated from the piston 10. Further sealing means 36 are provided between the piston 10 and the cylinder 6. In this embodiment in the inactivated position of the device the piston 10 is arranged in relation to the cylinder in such an manner that the dispersing chamber has its largest volume, i.e. the piston and the cylinder are in the retracted position in relation to each other. When the device is activated for inhalation a dose is placed in the inhalation channel by the rotating movement of the manoeuvring unit 13, which in this embodiment is handled directly by the user. The piston 10 is then moved within the cylinder 6, i.e. the piston is pushed towards the outlet of the cylinder 6, against the force of the biasing element 32. The volume of the dispersing chamber is decreased. The piston 10 is then released and will due to the force of the biasing element travel instantly towards the bottom of the cylinder thereby creating a negative pressure or vacuum in the dispersing chamber as the volume increases. The dose will be sucked with the air entering into the air inlets of the inhaler to the inhalation channel and further up to the dispersing chamber. When the pressure has been compensated the valve 18 will close and the user can inhale through the mouth piece as described above.

Further sealing means could be provided in order to secure an airtight sealing between the inhaler 12, the piston 10 and the cylinder 6.

The biasing element is preferably a spiral spring but any other type of resilient element could be used.

The use of the device to force the dose to be inhaled down into the lungs of a patient as described above could of course also be used with a device constructed in accordance with the second embodiment of the invention.

The volume of the spacer could be varied due to requirements and needs, and a preferred maximum volume of the dispersing chamber is between 50 - 250 ml.

The different parts of the inhalation device are preferably made of plastics, metallized plastics or metal but other materials are also possible.

The present invention is preferably directed to the use of a pharmaceutically active substance in powdered form wherein the powder is dispersed into the inner chamber of the inhalation device in a finely divided form wherein the particles are smaller than 10µm, preferably smaller than 3µm.

### Possible modifications of the invention

The inhalation device according to the present invention could of course be modified within the scope of the appended claims.

In the preferred embodiment the means for generating the negative pressure or vacuum are two cylinder-formed elements provided telescopically in relation to each other. In the preferred embodiment the elements have a circular cross-section but any other form such as squared is possible.

In the second embodiment a further cylinder could be provided in the second element 6. Said further cylinder is arranged to be separately movable in relation to the second element 6, whereby the biasing means are provided in a space between the two cylinders.

## Claims

1. An inhalation device for inhalation of a pharmaceutically active substance mounted on an inhaler (12), said inhalation device comprising
- a first non-movable element (10) and a second movable element (6), said first non-movable element (10) being arranged in said second element (6) defining a dispersing chamber (20) therebetween;
- and valve means (18, 16) provided in said first non-movable element (10)
- said first non-movable element (10) being rigidly mounted on the air outlet of the inhaler (12) whereby said second element (6) will move in relation to both the first non-movable element (10) and the inhaler (12) when the device is activated for inhalation thereby creating a vacuum or negative pressure in said dispersing chamber (20) and whereby said valve means (16, 18) regulates the air flow through said inhalation device during inhalation.

2. Inhalation device according to claim 1 ,
**characterised** in that the first non-movable element (10) is fixed on the mouth piece (15) adjacent the air outlet (11) of the inhaler (12).

3. Inhalation device according to claim 1 or 2, **chara cterised** in that said first and second elements (10, 6) are substantially cylinder formed whereby the first non-movable element (10) is formed as a piston.

4. Inhalation device according to claim 1, 2 or 3 ,
**characterised** in that an air tight seal (8) is provided between the outer surface of the piston (10) and the inner surface of the second element (6).

5. Inhalation device according to any of claims 1 to 4,
**characterised** in that said first element (10) is formed as a piston having an opening (26) and being provided on and surrounding the air outlet of the inhaler (12), whereby an air tight seal is provided between the piston and the air outlet of the inhaler.

6. Inhalation device according to claim 5,
**characterised** in that said opening of the piston is provided in direct alignment with the opening of the air outlet of the inhaler (12).

7. Inhalation device according to claim 5 or 6,
**characterised** in that said valve means comprises first valve means (18) provided to close the opening of the piston (10), said first valve means (18) being provided to open when negative pressure or vacuum is created in said dispersing chamber (20) and air is sucked through the inhaler to said chamber.

8. Inhalation device according to any of the preceding claims,
**characterised** in that said valve means further comprises second valve means (16) provided to close the opening or air outlet (22) of the second element (6), said second valve means (16) being provided to open when a user inhales through said air outlet (22).

9. Inhalation device according to claims 7 or 8,
**characterised** in that said valve means (16, 18) are formed as thin membranes rigidly attached at their one end to the walls of the first and second elements respectively and freely movable at their other end.

10. Inhalation device according to any of the preceding claims,
**characterised** in that inhaler is a conventional dry-powder inhaler.

11. Inhalation device according to claim 10,
**characterised** in that said inhaler comprises manoeuvring means (13), said means comprising a dosing unit and a dosing disc for feeding a dose of the pharmaceutically active powdered substance from a loading position to a position for inhalation in the inhalation channel of the inhaler.

12. Inhalation device according claim 11,
**characterised** in that said inhaler is a breath-actuated dry-powder inhaler, preferably a breath-actuated dry-powder inhaler sold under name Turbuhaler®.

13. Inhalation device according to any of the preceding claims,
**characterised** in that the piston (10) is rigidly mounted on the mouth piece 15 of the inhaler (12).

14. Inhalation device according to any of the preceding claims,
**characterised** in that a further substantially cylinder formed element (4) is provided around the outer surface of the first cylinder formed element (6) and being rotatable relative to said second element (6), said further cylinder formed element (4) having mounting means (24) in its lower part for mounting the inhaler in said element (4).

15. Inhalation device according to claim 14,
**characterised** in that the manoeuvring means (13) of the inhaler are fixedly provided in the mounting means of the further cylinder formed element (4) so that a rotation of the further cylinder formed element (4) in relation to the second element (6) will rotate the manoeuvring means of the inhaler and activate the inhaler for inhalation.

16. Inhalation device according to claims 1 to 12,
**characterised** in that biasing means (32) are provided between the piston (10) and the second element (6) forcing the second element move in relation to the piston from a retracted position to an expanded position.

17. Inhalation device according to claims 16,
**characterised** in that said biasing means (26) are a spiral spring.

18. Method of dispersing a pharmaceutically active substance in a dispersing chamber (20) by creating a negative pressure or vacuum in said dispersing chamber by using an inhalation device as described in claims 1 to 15.

19. Method according to claim 18, whereby the active substance may be sucked through the air outlet of the inhaler through the dispersing chamber whereby the inhalation air flow is regulated by the valve means (16, 22)

20. Method of administering a dose of a pharmaceutically active powdered substance by using an inhalation device according to claims 1 to 17 whereby the device is also used to force the dose in the dispersing chamber (20) out through the air outlet (22) of the device.

21. Method according to claim 20, wherein the piston (10) and the second element (6) are forced to move in a direction opposite to the direction of creating said negative pressure or vacuum in the dispersing chamber whereby an over pressure or positive pressure will be created in the dispersing chamber forcing the dose out of the device.

## Patentansprüche

1. Inhaliervorrichtung zum Inhalieren eines pharmazeutischen Wirkstoffs, die an einem Inhalator (12) angebracht ist, wobei die Inhaliervorrichtung folgendes umfaßt:
- ein erstes nichtbewegliches Element (10) und ein zweites bewegliches Element (6), wobei das erste nichtbewegliche Element (10) in dem zweiten Element (6) angeordnet ist und dazwischen eine Dispersionskammer (20) definiert,
- und in dem ersten nichtbeweglichen Element (10) vorgesehene Ventilmittel (18, 16),
- wobei das erste nichtbewegliche Element (10) am Luftauslaß des Inhalators (12) starr angebracht ist, wodurch sich das zweite Element (6) bezüglich des ersten nichtbeweglichen Elements (10) und des Inhalators (12) bewegt, wenn die Vorrichtung zur Inhalation aktiviert wird, wodurch ein Vakuum oder Unterdruck in der Dispersionskammer (20) erzeugt wird und wodurch das Ventilmittel (16, 18) bei der Inhalation den Luftstrom durch die Inhaliervorrichtung reguliert.

2. Inhaliervorrichtung nach Anspruch 1, dadurch gekennzeichnet, daß das erste nichtbewegliche Element (10) am Mundstück (15) neben dem Luftauslaß (11) des Inhalators (12) befestigt ist.

3. Inhaliervorrichtung nach Anspruch 1 oder 2, dadurch gekennzeichnet, daß das erste und das zweite Element (10, 6) im wesentlichen zylinderförmig sind, wobei das erste nichtbewegliche Element (10) als Kolben ausgebildet ist.

4. Inhaliervorrichtung nach Anspruch 1, 2 oder 3, dadurch gekennzeichnet, daß eine luftdichte Dichtung (8) zwischen der Außenfläche des Kolbens (10) und der Innenfläche des zweiten Elements (6) vorgesehen ist.

5. Inhaliervorrichtung nach einem der Ansprüche 1 bis 4, dadurch gekennzeichnet, daß das erste Element (10) als ein Kolben mit einer Öffnung (26), der am Luftauslaß des Inhalators (12) vorgesehen ist und diesen umgibt, ausgebildet ist, wobei eine luftdichte Dichtung zwischen dem Kolben und dem Luftauslaß des Inhalators vorgesehen ist.

6. Inhaliervorrichtung nach Anspruch 5, dadurch gekennzeichnet, daß die Öffnung des Kolbens in direkter Ausrichtung auf die Öffnung des Luftauslasses des Inhalators (12) vorgesehen ist.

7. Inhaliervorrichtung nach Anspruch 5 oder 6, dadurch gekennzeichnet, daß das Ventilmittel ein erstes Ventilmittel (18), das zum Schließen der Öffnung des Kolbens (10) vorgesehen ist, umfaßt, wobei das erste Ventilmittel (18) so ausgeführt ist, daß es sich öffnet, wenn Unterdruck oder Vakuum in der Dispersionskammer (20) erzeugt und Luft durch den Inhalator zur Kammer gesaugt wird.

8. Inhaliervorrichtung nach einem der vorhergehenden Ansprüche, dadurch gekennzeichnet, daß das Ventilmittel weiterhin ein zweites Ventilmittel (16) umfaßt, das zum Schließen der Öffnung oder des Luftauslasses (22) des zweiten Elements (6) vorgesehen ist, wobei das zweite Ventilmittel (16) so ausgeführt ist, daß es sich öffnet, wenn ein Benutzer durch den Luftauslaß (22) inhaliert.

9. Inhaliervorrichtung nach Anspruch 7 oder 8, dadurch gekennzeichnet, daß die Ventilmittel (16, 18) als dünne Membranen ausgebildet sind, die an ihrem einen Ende starr an den Wänden des ersten bzw. des zweiten Elements befestigt und an ihrem anderen Ende frei beweglich sind.

10. Inhaliervorrichtung nach einem der vorhergehenden Ansprüche, dadurch gekennzeichnet, daß der Inhalator ein herkömmlicher Trockenpulverinhalator ist.

11. Inhaliervorrichtung nach Anspruch 10, dadurch gekennzeichnet, daß der Inhalator ein Betätigungsmittel (13) umfaßt, das eine Dosiereinheit und eine Dosierscheibe zum Zuführen einer Dosis des pharmazeutischen pulverförmigen Wirkstoffs von einer Ladeposition zu einer Position zum Inhalieren im Inhalationskanal des Inhalators umfaßt.

12. Inhaliervorrichtung nach Anspruch 11, dadurch gekennzeichnet, daß der Inhalator ein atmungsbetätigter Trockenpulverinhalator, vorzugsweise ein unter dem Namen Turbuhaler® vertriebener atmungsbetätigter Trockenpulverinhalator, ist.

13. Inhaliervorrichtung nach einem der vorhergehenden Ansprüche, dadurch gekennzeichnet, daß der Kolben (10) am Mundstück (15) des Inhalators (12) starr angebracht ist.

14. Inhaliervorrichtung nach einem der vorhergehenden Ansprüche, dadurch gekennzeichnet, daß ein weiteres im wesentlichen zylinderförmiges Element (4) um die Außenfläche des ersten zylinderförmigen Elements (6) herum vorgesehen und bezüglich des zweiten Elements (6) drehbar ist, wobei das weitere zylinderförmige Element (4) in seinem unteren Teil ein Befestigungsmittel (24) zur Befestigung des Inhalators in dem Element (4) aufweist.

15. Inhaliervorrichtung nach Anspruch 14, dadurch gekennzeichnet, daß das Betätigungsmittel (13) des Inhalators im Befestigungsmittel des weiteren zylinderförmigen Elements (4) fest vorgesehen ist, so daß eine Drehung des weiteren zylinderförmigen Elements (4) bezüglich des zweiten Elements (6) das Betätigungsmittel des Inhalators dreht und den Inhalator zur Inhalation aktiviert.

16. Inhaliervorrichtung nach den Ansprüchen 1 bis 12, dadurch gekennzeichnet, daß Vorspannmittel (32) zwischen dem Kolben (10) und dem zweiten Element (6) vorgesehen sind, die das zweite Element zur Bewegung bezüglich des Kolbens aus einer zurückgezogenen Position in eine ausgefahrene Position zwingen.

17. Inhaliervorrichtung nach Anspruch 16, dadurch gekennzeichnet, daß die Vorspannmittel (32) eine Spiralfeder sind.

18. Verfahren zur Dispersion eines pharmazeutischen Wirkstoffs in einer Dispersionskammer (20) durch Erzeugen eines Unterdrucks oder eines Vakuums in der Dispersionskammer unter Verwendung einer Inhaliervorrichtung wie in den Ansprüchen 1 bis 15 beschrieben.

19. Verfahren nach Anspruch 18, durch das der Wirkstoff durch den Luftauslaß des Inhalators durch die Dispersionskammer gesaugt werden kann, wobei der Inhalationsluftstrom durch die Ventilmittel (16, 18) reguliert wird.

20. Verfahren zur Verabreichung einer Dosis eines pharmazeutischen pulverförmigen Wirkstoffs durch Verwendung einer Inhaliervorrichtung nach den Ansprüchen 1 bis 17, wobei die Vorrichtung auch dazu verwendet wird, die Dosis in der Dispersionskammer (20) durch den Luftauslaß (22) der Vorrichtung nach draußen zu zwingen.

21. Verfahren nach Anspruch 20, bei dem der Kolben (10) und das zweite Element (6) zur Bewegung in einer der Richtung der Erzeugung des Unterdrucks oder des Vakuums in der Dispersionskammer entgegengesetzten Richtung gezwungen werden, wobei ein Überdruck in der Dispersionskammer erzeugt wird, der die Dosis aus der Vorrichtung hinauszwingt.

## Revendications

1. Appareil d'inhalation pour l'inhalation d'une substance pharmaceutiquement active fixé sur un inhalateur (12), ledit appareil d'inhalation comprenant
- un premier élément non-déplaçable (10) et un deuxième élément déplaçable (6), ledit premier élément non-déplaçable (10) étant disposé dans ledit deuxième élément (6) définissant une chambre de dispersion (20) entre eux ;
- et un moyen de valve (18, 16) prévu dans ledit premier élément non-déplaçable (10)
- ledit permier élément non-déplaçable (10) étant fixé rigidement sur la sortie d'air de l'inhalateur (12), ledit deuxième élément (6) se déplaçant ainsi par rapport au premier élément non-déplaçable (10) et aussi par rapport à l'inhalateur (12) lorsque l'appareil est activé pour l'inhalation, en créant ainsi un vide ou une pression négative dans ladite chambre de dispersion (20) et ledit moyen de valve (16, 18) régulant ainsi le flux d'air à travers ledit appareil d'inhalation au cours de l'inhalation.

2. Appareil d'inhalation selon la revendication 1, caractérisé en ce que ledit premier élément non-déplaçable (10) est fixé sur l'embout (15) adjacent à la sortie d'air (11) de l'inhalateur (12).

3. Appareil d'inhalation selon la revendication 1 ou 2, caractérisé en ce que lesdits premier et deuxième éléments (10, 6) sont substantiellement de forme cylindrique, le premier élément non-déplaçable (10) étant ainsi formé en tant que piston.

4. Appareil d'inhalation selon la revendication 1, 2 ou 3, caractérisé en ce qu'un joint étanche à l'air (8) est prévu entre la surface extérieure du piston (10) et la surface intérieure du deuxième élément (6).

5. Appareil d'inhalation selon l'une quelconque des revendications 1 à 4, caractérisé en ce que ledit premier élément (10) est formé en tant que piston ayant une ouverture (26) et étant prévu sur et entourant la sortie d'air de l'inhalateur (12), un joint étanche à l'air étant prévu entre le piston et la sortie d'air de l'inhalateur.

6. Appareil d'inhalation selon la revendication 5, caractérisé en ce que ladite ouverture du piston est prévue en alignement direct avec l'ouverture de la sortie d'air de l'inhalateur (12).

7. Appareil d'inhalation selon la revendication 5 ou 6, caractérisé en ce que ledit moyen de valve comprend un premier moyen de valve (18) prévu pour fermer l'ouverture du piston (10), ledit premier moyen de valve (18) étant prévu pour s'ouvrir lorsque la pression négative ou le vide est créé dans ladite chambre de dispersion (20) et que de l'air est aspiré à travers l'inhalateur dans ladite chambre.

8. Appareil d'inhalation selon l'une quelconque des revendications précédentes, caractérisé en ce que ledit moyen de valve comprend en outre un deuxième moyen de valve (16) prévu pour fermer l'ouverture ou la sortie d'air (22) du deuxième élément (6), ledit deuxième moyen de valve (16) étant prévu pour s'ouvrir lorsqu'un utilisateur inspire à travers ladite sortie d'air (22).

9. Appareil d'inhalation selon les revendications 7 ou 8, caractérisé en ce que lesdits moyens de valve (16, 18) sont formés en tant que minces membranes attachées rigidement à l'une de leurs extrémités aux parois des premier et deuxième éléments respectivement, et sont librement déplaçables au niveau de leur autre extrémité.

10. Appareil d'inhalation selon l'une quelconque des revendications précédentes, caractérisé en ce que l'inhalateur est un inhalateur conventionnel de poudre sèche.

11. Appareil d'inhalation selon la revendication 10, caractérisé en ce que ledit inhalateur comprend un moyen de manipulation (13), ledit moyen comprenant une unité de dosage et un disque de dosage pour fournir une dose de la substance poudreuse pharmaceutiquement active d'une position de chargement dans une position d'inhalation dans le canal d'inhalation de l'inhalateur.

12. Appareil d'inhalation selon la revendication 11, caractérisé en ce que ledit inhalateur est un inhalateur de poudre sèche à actionnement par la respiration, de préférence un inhalateur de poudre sèche à actionnement par la respiration vendu sous la dénomination Turbuhaler®.

13. Appareil d'inhalation selon l'une quelconque des revendications précédentes, caractérisé en ce que le piston (10) est fixé rigidement sur l'embout 15 de l'inhalateur (12).

14. Appareil d'inhalation selon l'une quelconque des revendications précédentes, caractérisé en ce qu'un élément supplémentaire (4) de forme substantiellement cylindrique est prévu autour de la surface extérieure du premier élément (6) de forme cylindrique et peut tourner par rapport audit deuxième élément (6), ledit élément supplémentaire (4) de forme cylindrique ayant un moyen de fixation (24) dans sa partie inférieure pour fixer l'inhalateur dans ledit élément (4).

15. Dispositif d'inhalation selon la revendication 14, caractérisé en ce que le moyen de manipulation (13) de l'inhalateur est prévu fixement dans le moyen de fixation de l'élément supplémentaire (4) de forme cylindrique de sorte qu'une rotation de l'élément supplémentaire (4) de forme cylindrique par rapport au deuxième élément (6) fera tourner le moyen de manipulation de l'inhalateur et activera l'inhalateur en vue de l'inhalation.

16. Appareil d'inhalation selon les revendications 1 à 12, caractérisé en ce que des moyens de poussée (32) sont prévus entre le piston (10) et le deuxième élément (6), pour forcer le deuxième élément à se déplacer par rapport au piston depuis une position rétractée dans une position déployée.

17. Appareil d'inhalation selon la revendication 16, caractérisé en ce que lesdits moyens de poussée (26) sont un ressort spiral.

18. Méthode de dispersion d'une substance pharmaceutiquement active dans une chambre de dispersion (20) en créant une pression négative ou un vide dans ladite chambre de dispersion en utilisant un appareil d'inhalation selon les revendications 1 à 15.

19. Méthode selon la revendication 18, dans laquelle la substance active peut être aspirée à travers la sortie d'air de l'inhalateur par la chambre de dispersion, le flux d'air d'inhalation étant régulé par les moyens de valve (16, 18).

20. Méthode d'administration d'une dose d'une substance poudreuse pharmaceutiquement active en utilisant un appareil d'inhalation selon les revendications 1 à 17, dans laquelle l'appareil est également utilisé pour forcer la dose dans la chambre de dispersion (20) vers l'extérieur à travers la sortie d'air (22) de l'appareil.

21. Méthode selon la revendication 20, dans laquelle le piston (10) et le deuxième élément (6) sont forcés de se déplacer dans une direction opposée à la direction de création de ladite pression négative ou dudit vide dans la chambre de dispersion, une surpression ou une pression positive étant créée dans la chambre de dispersion, forçant la dose hors de l'appareil.
